# EUROPÄISCHE PATENTSCHRIFT

(11) **EP 1 408 009 B1**
(45) Veröffentlichungstag und Bekanntmachung des Hinweises auf die Patenterteilung: **31.08.2005**
(21) Anmeldenummer: 02022760.9
(22) Anmeldetag: 11.10.2002
(51) Int. Cl.: C02F 3/28

(54) **Reaktor mit zwei Gasabscheidern und Verfahren zur anaeroben Behandlung von Flüssigkeiten**
Reactor with two gas separators and method for the anaerobic treatment of liquids
Réacteur à deux séparateurs de gaz et procédé anaérobie de traitement de liquides

(43) Veröffentlichungstag der Anmeldung: 14.04.2004
(73) Patentinhaber: VA TECH WABAG GmbH, 1211 Wien (AT)
(72) Erfinder: Datschewski, Peter, 42489 Wülfrath (DE); Risse, Henry, 52068 Aachen (DE); Klegraf, Ferdinand, 65345 Rauenthal (DE); Tippmann, Kurt, 35428 Langgöns (DE)
(74) Vertreter: VA TECH Patente GmbH & Co

(56) Entgegenhaltungen:
- EP-A- 0 300 348
- EP-A- 0 808 805
- DE-A- 4 213 015
- US-A- 4 609 460
- PATENT ABSTRACTS OF JAPAN vol. 010, no. 383 (C-393), 23. Dezember 1986 (1986-12-23) & JP 61 174995 A (NIPPON BEET SUGAR MFG CO LTD;OTHERS: 01), 6. August 1986 (1986-08-06)

## Beschreibung

Die Erfindung betrifft einen Reaktor sowie ein entsprechendes Verfahren zur anaeroben Behandlung von Flüssigkeiten, inbesondere von Abwasser oder wässrigen Prozesslösungen, durch anaerobe biologische Umsetzung organischer Verunreinigungen mittels granulierter, flockiger oder auf leicht fluidisierbaren Trägermaterialien fixierter Biomasse, wobei der Reaktor einen ersten Gasabscheider und einen zweiten Gasabscheider in einem Abstand darüber aufweist.

Die anaerobe Reinigung organisch belasteter Abwässer wird vorteilhaft in Schlammbettreaktoren betrieben, die nach dem UASB-Prinzip arbeiten. Reaktoren, die nach diesem Prinzip arbeiten, werden z.B. in der EP 0 244 029 B1 und EP 0 808 805 A1 beschrieben. Auch die hier beschriebene Erfindung nutzt das UASB-Prinzip.

Der Vorgang der Reinigung läuft in einem Dreiphasensystem ab, bestehend aus der Flüssigkeit, welche das zu behandelnde Abwasser darstellt, aus dem Feststoff - der im Schlammbett vorliegenden flockigen oder granulierten Biomasse oder dem leicht fluidisierbaren Biomasseträger - sowie der Gasphase, die vorwiegend aus Methan CH₄, Kohlendioxid CO₂ und Spurengasen besteht.

Die Biomasse eines UASB-Reaktors liegt idealerweise in Form eines Gemisches (Schlamm) vor, dass sich aus kugelförmigen Agglomeraten (Schlammpellets) und Feinschlamm zusammensetzt, wobei die Schlammpellets idealer Weise Durchmesser von unter 1 bis 5 mm und eine höhere Dichte als Wasser aufweisen, wodurch sich ein Schlammbett ausbildet. Die Biomasse kann aber auch auf Trägermedien aufgebracht werden, sodass sich die folgenden Ausführungen zu Schlamm bzw. Schlammpellets auch auf diese mit Biomasse bewachsenen Trägermedien beziehen.

Im Schlammbett erfolgt ein Großteil der Umsetzung des organischen Materials. Durch die im Schlammbett erfolgende Biogaserzeugung kommt es zu einer Blasenbildung. Die aufsteigenden Blasen und der aufwärts gerichtete Flüssigkeitsstrom fluidisieren das Schlammbett.

UASB-Reaktoren enthalten Dreiphasen-Trennvorrichtungen, mit denen die drei Phasen Biogas, gereinigtes Abwasser und Biomasse voneinander getrennt werden. Diese Dreiphasen-Trennvorrichtungen sind in der Regel über dem Schlammbett angeordnet, enthalten eine oder mehrere Hauben zum Auffangen und Fortleiten des Biogases sowie an der Wasseroberfläche Ablaufrinnen für die Fortleitung des gereinigten Abwassers. Diese Konstruktionen erlauben nach Gasabtrennung die Sedimentation der Biomasse.

Der auf diese Weise konstruierte UASB-Reaktor ist großvolumig und erlaubt nur geringe Umsatzraten. Weiters ist aus der US 4 609 460 A bekannt, zwei Gasabscheider jeweils bestehend aus mehreren übereinander angeordneten Hauben übereinander anzuordnen, wobei jeder Gasabscheider ein eigenes Rohr zum Ableiten von abgetrenntem Gas aufweist.

Eine Aufgabe der Erfindung ist es nun, den Reaktoraufbau so zu modifizieren, dass sehr hohe volumenspezifische Umsatzraten und damit eine effizientere Ausnutzung des Reaktorvolumens erzielt werden können.

Diese Aufgaben werden erfindungsgemäß durch einen Reaktor nach Anspruch 1 bzw. durch ein Verfahren nach Anspruch 11 gelöst. Die Ansprüche 2 bis 10 betreffen bevorzugte Ausführungen der Erfindung.

Die Biomasse bildet im nicht fluidisiertem Zustand ein Schlammbett, in dessen Bodenbereich das zu behandelnde Abwasser zugeführt wird. Der erste Gasabscheider ist im unteren Bereich des Reaktors angeordnet, also im Schlammbett des Reaktors. Im Schlammbett erfolgt ein Großteil der biochemischen Umsetzung des organischen Materials. Das dabei entstehende Biogas wird zu einem erheblichen Teil vom ersten Gasabscheider gefasst und in ein zentrales Steigrohr geleitet. Das restliche Gas durchströmt den Bereich über dem Schlammbett und wird in einem zweiten Gasabscheider gefasst, der sich über dem Schlammbett befindet und ebenfalls in das Steigrohr abgeleitet. Ein geringer Rest entweicht an der Wasseroberfläche in den darüberliegenden Gassammelraum. Oberhalb des zweiten Gasabscheiders befindet sich eine Sedimentationszone für die Biomasse.

Durch einen im Schlammbett des Reaktors angeordneten Gasabscheider wird ein Teil des Biogases schon im Schlammbett aus dem System entnommen, wodurch zu hohe Gasleerrohrgeschwindigkeiten vermieden werden.

Der Durchmesser des Steigrohres ist dabei so bemessen, dass 5 bis 25% der Querschnittsfläche des Reaktors vom Steigrohr ausgefüllt wird. Dadurch wird in der Regel ein Gashebereffekt (Mammutpumpeneffekt) vermieden.

Indem das Steigrohr etwa in einer zentralen Achse des Reaktors, insbesondere in seiner zentralen Längsachse, angeordnet ist, können im Reaktor zu dieser zentralen Achse symmetrische Strömungsbedingungen hergestellt werden.

Es ist konstruktiv einfach, wenn ein Gasabscheider aus gleichartigen Gasabscheiderelementen aufgebaut ist. Dabei ist es ausreichend, wenn die Gasabscheider jeweils aus einer Lage Gasabscheiderelementen gebildet werden.

Vorteilhaft ist, wenn die Gasabscheiderelemente radial zum Steigrohr angeordnet sind, weil dann das auf der konkaven Unterseite des Gasabscheiderelements gesammelte Gas dem Gasabscheiderelement entlang zum Steigrohr strömen kann. Dies kann dadurch unterstützt werden, dass die Gasabscheiderelemente zur Horizontalen schräg angeordnet sind, also deren Ende an der Reaktorwand tiefer liegt als jenes beim Steigrohr.

Vorteilhaft und einfach ist auch die Ausgestaltung, nach welcher das Steigrohr für jedes Gasabscheiderelement zumindest eine Gasübertrittsöffnung aufweist, von welcher das durch das Gasabscheiderelement gesammelte Gas direkt in das Steigrohr strömen kann. Es sind somit keine weiteren Einrichtungen, wie ein Ableitungsrohr für jedes einzelne Gasabscheiderelement vonnöten, um das Gas abzuleiten. Diese Funktion wird allein durch das Gasabscheiderelement und die Gasübertrittsöffnung erfüllt.

Etwaige in das Steigrohr mitgeführte Biomasse kann auf einfache Weise im Steigrohr selbst nach unten befördert werden, indem dieses Öffnungen aufweist, durch welche Biomasse aus dem Steigrohr in den Reaktorraum absinken kann. Der mitgeführte Schwimmschlamm wird am oberen Ende des Steigrohres durch eine Tauchwand abgeschieden. Die außerhalb der Tauchwand angeordneten Abzugseinrichtungen werden auf diese Weise von Schwimmschlamm freigehalten.

Nachfolgend wird die Erfindung anhand der als Beispiele angeführten schematischen Fig. 1-3 näher erläutert. Es zeigen:
- Fig. 1:: einen Längsschnitt des erfindungsgemäßen Reaktors
- Fig. 2:: einen Querschnitt des erfindungsgemäßen Reaktors
- Fig. 3:: eine Detaildarstellung der Gasabscheiderelemente

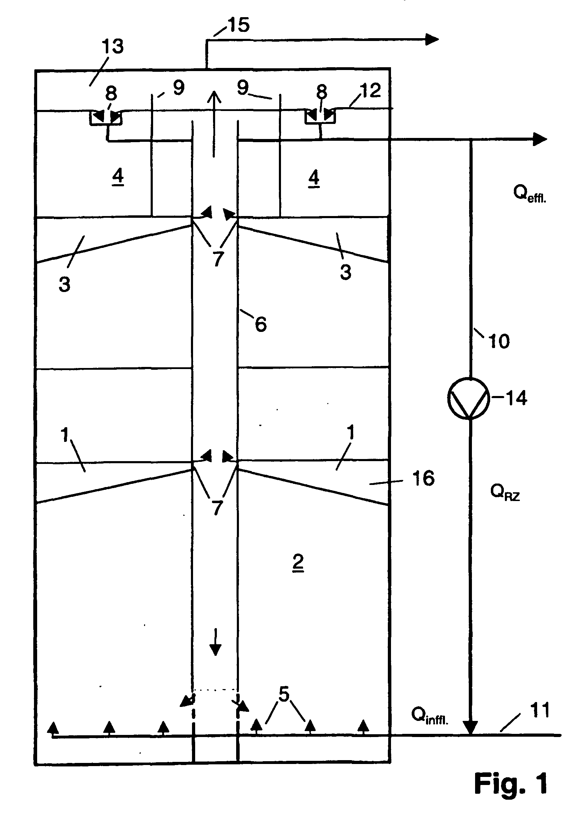

Der Reaktor gemäß Fig. 1 umfasst die Einspeisestellen 5 für das Abwasser, einen ersten Gasabscheider 1 im Reaktionsraum innerhalb des Schlammbettes 2 (grau dargestellt) sowie einen zweiten Gasabscheider 3 oberhalb des Schlammbettes 2 sowie eine Sedimentationszone 4 im Reaktorkopf. Das zu behandelnde Abwasser wird über eine Zuführrohrleitung 11 und die im Bodenbereich befindlichen Einspeisestellen 5 in das Schlammbett 2 eingespeist. In der unteren Hälfte des Reaktors befindet sich der erste Gasabscheider 1, welcher aus einer Lage von Gasabscheiderelementen besteht, die das Auffangen des gebildeten Biogases ermöglichen und es in Richtung des zentralen Steigrohres 6 fortleiten, in welchem das Biogas aufsteigt. Über dem Schlammbett 2 nahe der Wasseroberfläche 12 befindet sich ein zweiter Gasabscheider 3, der ebenfalls aus einer Lage von Gasabscheiderelementen besteht.

Die Verbindung zwischen den Gasabscheiderelementen der beiden Gasabscheider 1, 3 und dem geraden, zentralen Steigrohr 6 bilden Gaseintrittsöffnungen 7, die den Übertritt des Biogases in das Steigrohr 6 ermöglichen, sodass das Biogas in den Gassammelraum 13 aufsteigen kann. Von dort wird das Biogas, wie durch Leitung 15 angedeutet, abgezogen.

Durch die Gasvorabscheidung im Schlammbett 2 durch den ersten Gasabscheider 1 und durch den zweiten Gasabscheider 3 am Reaktorkopf wird die Sedimentation der Biomasse, vorzugsweise Schlammpellets, im Sedimentationsraum 4 über dem zweiten Gasabscheider 3 erreicht. An der Wasseroberfläche befinden sich Klarwasserabzugseinrichtungen 8, wie etwa Ablaufrinnen. Zur Abscheidung bzw. Rückhaltung von Schwimmschlamm von den Abzugseinrichtungen ist das zentrale Steigrohr 6 mit einer Tauchwand 9 umgeben. Die Tauchwand 9 ist hier als Rohr ausgebildet, das konzentrisch zum Steigrohr 6 außerhalb des Steigrohrs angeordnet ist, und reicht vom zweiten Gasabscheider 3 bis über das obere Ende des Steigrohres 6, welches unter der Wasseroberfläche 12 endet und weiter über die Wasseroberfläche hinaus in den Gassammelraum 13.

Das Steigrohr weist im unteren Bereich nahe dem Boden Öffnungen 20 auf, durch welche Biomasse aus dem Steigrohr 6 zurück in den Reaktorraum absinken kann.

Das Steigrohr 6 reicht bis zum Boden des Reaktors, wobei das untere Rohrende gegenüber dem Reaktor abgeschlossen ist. Das obere Rohrende ist des Steigrohres ist offen.

Zur Einstellung definierter Zulaufkonzentrationen ist ggf. eine Rezirkulation vom Ablauf des Reaktors über eine Pumpe 14 und eine Rezirkulationsleitung 10 in die Zuführrohrleitung 11 erforderlich.

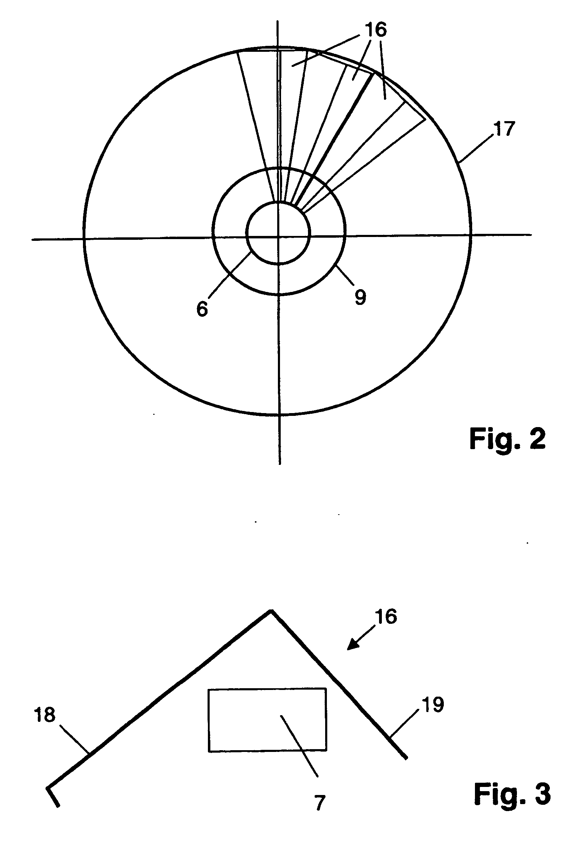

Fig. 2 ist ein Querschnitt gemäß einer horizontalen Schnittfläche zwischen zweitem Gasabscheider 3 und Wasseroberfläche 12 gemäß Fig. 1 und zeigt die Anordnung der Gasabscheiderelemente 16 im Reaktor, wobei nur drei Gasabscheiderelemente 16 dargestellt sind. Jeder Gasabscheider 1, 3 ist jedoch aus einer Lage von Gasabscheiderelementen 16 gebildet, die sich gegenseitig in Teilbereichen überdecken und im Wesentlichen den gesamten Reaktorquerschnitt außerhalb des Steigrohres 6 bedecken. Jedes Gasabscheiderelement 16 deckt einen sektorförmigen Querschnittsbereich des in diesem Fall kreisrunden Reaktorquerschnitts ab und reicht von der Wand des Steigrohres 6 bis zur Reaktorwand 17.

Fig. 3 zeigt ein Gasabscheiderelement 16 im Querschnitt mit Blickrichtung zum Steigrohr 6. Der Querschnitt des Gasabscheiderelements 16 ist winkelförmig und asymmetrisch ausgebildet, indem ein Schenkel 18 des haubenförmigen Gasabscheiderelements länger ausgebildet ist als der andere Schenkel 19. Der Querschnitt der Gasabscheiderelemente 16 vergrößert sich in radialer Richtung proportional zum Abstand vom Steigrohr 6, sodass - wie in Fig. 2 dargestellt - der Reaktorquerschnitt im Wesentlichen durch eine Lage von Gasabscheiderelementen 16 abgedeckt werden kann.

Die im Sedimentationsraum 4 sedimentierten Schlammpellets gleiten entlang der Oberseiten der Gasabscheiderelemente 16 und sinken zwischen den Gasabscheiderelementen 16 nach unten zurück in das Schlammbett.

## Patentansprüche

1. Reaktor zur anaeroben Behandlung von Flüssigkeiten, inbesondere von Abwasser oder wässrigen Prozesslösungen, durch anaerobe biologische Umsetzung organischer Verunreinigungen mittels granulierter, flockiger oder auf leicht fluidisierbaren Trägermaterialien fixierter Biomasse, wobei der Reaktor einen ersten Gasabscheider (1) und einen zweiten Gasabscheider (3) in einem Abstand darüber aufweist und im Reaktor zum Ableiten des erzeugten Biogases aus den beiden Gasabscheidem (1, 3) ein Steigrohr (6) angeordnet ist, welches die beiden Gasabscheider gasseitig miteinander verbindet, **dadurch gekennzeichnet, dass** der Durchmesser des Steigrohres (6) zur Vermeidung von Mammutpumpeneffekten so bemessen ist, dass 5 bis 25% der Querschnittsfläche des Reaktors vom Steigrohr ausgefüllt werden und das Steigrohr (6) unter der Wasseroberfläche (12) endet.

2. Reaktor nach Anspruch 1, **dadurch gekennzeichnet, dass** Gasabscheider (1, 3) und Steigrohr (6) so angeordnet sind, dass das in den Gasabscheidem (1, 3) gesammelte Gas jeweils auf der Höhe des Gasabscheiders (1, 3) direkt in dieses Steigrohr (6) geleitet wird.

3. Reaktor nach einem der Ansprüche 1 oder 2, **dadurch gekennzeichnet, dass** zumindest einer der Gasabscheider (1, 3) aus einzelnen gleichartigen, insbesondere in nur einer Lage angeordneten, Gasabscheiderelementen (16) gebildet wird.

4. Reaktor nach Anspruch 3, **dadurch gekennzeichnet, dass** mindestens einer der Gasabscheider (1, 3) radial zum Steigrohr (6) angeordnete Gasabscheiderelemente (16) aufweist.

5. Reaktor nach einem der Ansprüche 3 oder 4, **dadurch gekennzeichnet, dass** das der Reaktorwand nähere Ende der Gasabscheiderelemente (16) tiefer liegt als das dem Steigrohr (6) nähere Ende, sodass das gesammelte Gas den Gasabscheiderelementen (16) entlang zum Steigrohr (6) strömen kann.

6. Reaktor nach einem der Ansprüche 3 bis 5, **dadurch gekennzeichnet, dass** die Gasabscheiderelemente (16) relativ zur Fläche, die senkrecht durch die Scheitellinie der Gasabscheiderelemente verläuft, einen asymmetrischen Querschnitt (18, 19) aufweisen.

7. Reaktor nach einem der Ansprüche 3 bis 6, **dadurch gekennzeichnet, dass** das Steigrohr (6) für jedes Gasabscheiderelement (16) zumindest eine Gasübertrittsöffnung (7) aufweist, durch welche das durch das Gasabscheiderelement (16) gesammelte Gas direkt in das Steigrohr (6) strömen kann.

8. Reaktor nach einem der Ansprüche 1 bis 7, **dadurch gekennzeichnet, dass** das Steigrohr (6) im Bereich unterhalb des ersten Gasabscheiders (1) Öffnungen (20) aufweist, durch welche Biomasse aus dem Steigrohr (6) in den Reaktorraum absinken kann.

9. Reaktor nach einem der Ansprüche 1 bis 8, **dadurch gekennzeichnet, dass** das Steigrohr (6) an seinem oberen Ende zur Abscheidung von Schwimmschlamm von einer Tauchwand (9) umgeben ist.

10. Reaktor nach Anspruch 9, **dadurch gekennzeichnet, dass** zwischen der Tauchwand (9) und der Reaktorwand (17) Abzugseinrichtungen (8) für die gereinigte Flüssigkeit vorgesehen sind.

11. Verfahren zur Trennung eines Dreiphasengemisches bei der anaeroben Behandlung von Flüssigkeiten, inbesondere von Abwasser oder wässrigen Prozesslösungen, durch anaerobe biologische Umsetzung organischer Verunreinigungen mittels granulierter, flockiger oder auf leicht fluidisierbaren Trägermaterialien fixierter Biomasse, in einem Reaktor mit einem ersten (1) und einem in einem Abstand darüber angeordneten zweiten (3) Gasabscheider, wobei das in den Gasabscheidern (1, 3) gesammelte Gas auf der jeweiligen Ebene des Gasabscheiders (1, 3) in ein gemeinsames Steigrohr (6) innerhalb des Reaktors, welches die beiden Gasabscheider gasseitig miteinander verbindet, und in diesem Steigrohr zum Reaktorkopf geleitet wird, **dadurch gekennzeichnet, dass** das Steigrohr zur Vermeidung von Mammutpumpeneffekten so bemessen ist, dass 5 bis 25% der Querschnittsfläche des Reaktors vom Steigrohr ausgefüllt werden und das Steigrohr (6) unter der Wasseroberfläche (12) endet.

## Claims

1. Reactor for the anaerobic treatment of liquids, in particular of waste water or aqueous process solutions, by anaerobic biological conversion of organic impurities by means of granulated biomass, flocculent biomass or biomass which is fixed on readily fluidizable carrier materials, the reactor having a first gas separator (1) and a second gas separator (3) at a distance above it, and whereas a riser (6), which connects the two gas separators to one another on the gas side, is arranged in the reactor for the purpose of discharging the biogas which is generated from the two gas separators (1, 3), **characterized in that** to avoid airlift pump effects the diameter of the riser (6) is dimensioned in a way that the riser takes up 5 to 25% of the cross-sectional area of the reactor, and that the riser (6) ends below the water surface (12).

2. Reactor according to Claim 1, **characterized in that** gas separators (1, 3) and riser (6) are arranged in such a way that the gas which has collected in the gas separator (1, 3) is in each case passed directly into this riser (6) at the level of the gas separator (1, 3).

3. Reactor according to one of Claims 1 or 2, **characterized in that** at least one of the gas separators (1, 3) is formed from individual gas separator elements (16) which are of the same type and in particular are arranged in just one layer.

4. Reactor according to Claim 3, **characterized in that** at least one of the gas separators (1, 3) has gas separator elements (16) arranged radially with respect to the riser (6).

5. Reactor according to one of Claims 3 or 4, **characterized in that** the end of the gas separator elements (16) which lies nearer to the reactor wall is at a lower level than the end located at the riser (6) so that collected gas can flow along the gas separator element to the riser.

6. Reactor according to one of Claims 3 to 5, **characterized in that**, with relation to a plane which is perpendicular to the apex line of the gas separator elements (16), the gas separator elements (16) have an asymetric cross section (18, 19).

7. Reactor according to one of Claims 3 to 6, **characterized in that** the riser (6), for each gas separator element (16), has at least one gas transfer opening (7), through which the gas which has been collected by the gas separator element (16) can flow directly into the riser (6).

8. Reactor according to one of Claims 1 to 7, **characterized in that** the riser (6), in the area below the first gas separator (1), has openings (20) through which biomass can sink out of the riser (6) into the reactor space.

9. Reactor according to one of Claims 1 to 8, **characterized in that** the riser (6) is surrounded at its upper end by a submerged wall (9) for the purpose of separating off floating sludge.

10. Reactor according to Claim 9, **characterized in that** extraction devices (8) for extracting the purified liquid are provided between the submerged wall (9) and the reactor wall (17).

11. Method for separating a three-phase mixture during the anaerobic treatment of liquids, in particular of waste water or aqueous process solutions, by anaerobic biological conversion of organic impurities by means of granulated biomass, flocculent biomass or biomass which is fixed on readily fluidizable carrier materials, in a reactor having a first gas separator (1) and a second gas separator (3) arranged at a distance above it, whereas gas which has collected in the gas separators (1, 3), at the corresponding plane of the gas separator (1, 3), is passed into a common riser (6) inside the reactor, which connects the two gas separators to one another on the gas side, and in this riser is passed to the reactor head, **characterized in that** to avoid airlift pump effects the diameter of the riser (6) is dimensioned in a way that the riser takes up 5 to 25% of the cross-sectional area of the reactor, and that the riser (6) ends below the water surface (12).

## Revendications

1. Réacteur pour le traitement anaérobie de liquides, en particulier d'eaux usées ou de solutions aqueuses de processus, par conversion biologique anaérobie d'impuretés organiques au moyen d'une biomasse granulée, floconneuse ou fixée sur des matériaux support aisément fluidisables, où le réacteur présente un premier séparateur à gaz (1) et un deuxième séparateur à gaz (3), placé à distance au-dessus, et dans le réacteur étant disposé un tube montant (6) pour évacuer le biogaz produit depuis les deux séparateurs à gaz (1, 3), tube montant reliant ensemble côté gaz les deux séparateurs à gaz, **caractérisé en ce que**, pour éviter des effets de pompes mammouth, le diamètre du tube montant (6) est dimensionné de manière que de 5 à 25 % de l'aire de la section transversale du réacteur soient remplis par le tube montant, et le tube montant (6) s'achevant au-dessous de la surface d'eau (12).

2. Réacteur selon la revendication 1, **caractérisé en ce que** le séparateur à gaz (1, 3) et le tube montant (6) sont disposés de manière que le gaz collecté dans le séparateur à gaz (1, 3) soit dirigé, à la hauteur du séparateur à gaz (1, 3), directement dans ce tube montant (6).

3. Réacteur selon l'une des revendications 1 ou 2, **caractérisé en ce qu'**au moins l'un des séparateurs à gaz (1, 3) est formé d'éléments de séparateur à gaz (16) individuels du même type, en particulier disposés en une seule couche.

4. Réacteur selon la revendication 3, **caractérisé en ce qu'**au moins l'un des séparateurs à gaz (1, 3) présente des éléments séparateurs à gaz (16) disposés radialement par rapport au tube montant (6).

5. Réacteur selon l'une des revendications 3 ou 4, **caractérisé en ce que** l'extrémité la plus proche de la paroi du réacteur, des éléments séparateurs à gaz (16) est située plus bas que l'extrémité plus proche du tube montant (6), de manière que le gaz collecté puisse s'écouler vers le tube montant (6), en évoluant le long des éléments séparateurs à gaz (16).

6. Réacteur selon l'une des revendications 3 à 5, **caractérisé en ce que** les éléments de séparateurs à gaz (16) présentent une section transversale (18, 19) asymétrique, qui s'étend perpendiculairement, par la ligne de sommet des éléments séparateurs à gaz.

7. Réacteur selon l'une des revendications 3 à 6, **caractérisé en ce que** le tube montant (6) présente, pour chaque élément séparateur à gaz (16), au moins une ouverture de passage de gaz (7), à travers laquelle le gaz, collecté au moyen de l'élément séparateur à gaz (16), peut s'écouler directement dans le tube montant (6).

8. Réacteur selon l'une des revendications 1 à 7, **caractérisé en ce que** le tube montant (6), dans la zone située au-dessus du séparateur à gaz (1), présente des ouvertures (20), à travers lesquelles de la biomasse, issue du tube montant (6), peut descendre dans l'enceinte de réacteur.

9. Réacteur selon l'une des revendications 1 à 8, **caractérisé en ce que**, à son extrémité supérieure, le tube montant (6) est entouré par une paroi plongeante (9), afin de séparer vis-à-vis de la boue flottante.

10. Réacteur selon la revendication 8, **caractérisé en ce que**, entre la paroi plongeante (9) et la paroi de réacteur (17), sont prévus des dispositifs d'extraction (8) pour le liquide épuré.

11. Procédé de séparation d'un mélange à trois phases dans le traitement anaérobie de liquides, en particulier d'eaux usées ou de solutions aqueuses de processus, par conversion biologique anaérobie d'impuretés organiques au moyen d'une biomasse granulée, floconneuse ou fixée sur des matériaux support aisément fluidisables, dans un réacteur avec un premier (1) et un deuxième (3) séparateurs à gaz, disposé à distance au-dessus, le gaz collecté dans les séparateurs à gaz (1, 3) étant guidé sur le plan respectif du séparateur à gaz (1, 3) en un tube montant (6) commun à l'intérieur du réacteur, reliant les deux séparateurs à gaz ensemble côté gaz, et guidé dans ce tube montant, vers la tête de réacteur, **caractérisé en ce que**, pour éviter des effets de pompes mammouth, le tube montant est dimensionné de manière à ce que de 5 à 25 % de l'aire de la section transversale du réacteur soient remplis par le tube montant et que le tube montant (6) s'achève au-dessous de la surface d'eau (12).
